Europäisches Patentamt

European Patent Office

Office européen des brevets

(11) Veröffentlichungsnummer: **0 269 044 B1**

(12) **EUROPÄISCHE PATENTSCHRIFT**

(45) Veröffentlichungstag der Patentschrift: **23.10.91**

(21) Anmeldenummer: **87117211.0**

(22) Anmeldetag: **23.11.87**

(51) Int. Cl.⁵: **C07C 67/48**, C07C 69/40,
C07D 213/06, C07B 63/00,
C07D 213/16

(54) **Verfahren zur Rückgewinnung von Pyridin oder Methylpyridinen oder deren Gemische aus Butandicarbonsäuremethylester enthaltenden Reaktionsgemischen.**

(30) Priorität: **25.11.86 DE 3640296**

(43) Veröffentlichungstag der Anmeldung:
**01.06.88 Patentblatt 88/22**

(45) Bekanntmachung des Hinweises auf die
Patenterteilung:
**23.10.91 Patentblatt 91/43**

(84) Benannte Vertragsstaaten:
**BE CH DE ES FR GB IT LI NL**

(56) Entgegenhaltungen:
**EP-A- 0 008 412**
**EP-A- 0 010 581**

**PATENT ABSTRACTS OF JAPAN, Band 9, Nr.
1 (C-259) (1724), 5. Januar 1985 & JP-A-59
155359**

(73) Patentinhaber: **BASF Aktiengesellschaft
Carl-Bosch-Strasse 38
W-6700 Ludwigshafen(DE)**

(72) Erfinder: **Lendle, Hubert, Dr.
Ruedigerstrasse 41
W-6700 Ludwigshafen(DE)**
Erfinder: **Panitz, Paul
Wachenheimer Strasse 1
W-6520 Worms 1(DE)**
Erfinder: **Seyfert, Wilfried, Dr.
Bachweg 8
W-6719 Weisenheim(DE)**
Erfinder: **Stops, Peter
Limburgstrasse 12
W-6701 Altrip(DE)**

## Beschreibung

Bei der Herstellung Von Butandicarbonsäuremethylestern durch Umsetzen von Pentensäuremethylestern mit Kohlenmonoxid und Methanol in Gegenwart von tertiären Stickstoffbasen, wie Pyridin oder Methylpyridinen sowie Kobaltcarbonylkatalysatoren und nachfolgender Behandlung mit molekularen Sauerstoff enthaltenden Gasen in wäßriger Essigsäure, wie es in der EP-Anmeldung 10 581 beschrieben wird, führt man wäßrige Kobaltacetatlösung wieder zur Katalysatorherstellung zurück, während man die organische Phase durch Destillation aufarbeitet. Es hat sich jedoch herausgestellt, daß die im Reaktionsgemisch enthaltenen tertiären Stickstoffbasen wie Pyridin oder Methylpyridine abgetrennt werden müssen, da sonst die anfallende Kobaltacetatlösung stark verunreinigt ist.

Sowohl nach dem in der EP-A-10 581 als auch in der EP-A-8412 beschriebenen Verfahren werden N-Basen aus der organischen Phase, die Adipinsäureester, andere Dicarbonsäureester und Nebenprodukte sowie Pentensäureester enthält, durch fraktionierte Destillation abgetrennt. Dies ist jedoch technisch sehr aufwendig, insbesondere im Hinblick auf die Bildung von Azeotropen. Desgleichen wird in der japanischen Offenlegungsschrift 84-155 359 die destillative Rückgewinnung von Pyridin aus Hydroesterifizierungsgemischen beschrieben, wobei die vorgenannten Schwierigkeiten auftreten.

Es war deshalb die technische Aufgabe gestellt, aus Butandicarbonsäuremethylester enthaltenden Reaktionsgemischen Pyridin, Methylpyridin oder deren Gemische in wiederverwendbarer Form zurückzugewinnen und eine für die Katalysatorherstellung geeignetere Kobaltacetatlösung zu erhalten.

Diese Aufgabe wird gelöst in einem Verfahren zur Rückgewinnung von Pyridin, Methylpyridinen oder deren Gemischen aus Butandicarbonsäuremethylester enthaltenden Reaktionsgemischen, die durch Umsetzung von Pentensäuremethylester mit Kohlenmonoxid und Methanol in Gegenwart von Pyridin, Methylpyridinen oder deren Gemischen sowie Kobaltcarbonylkatalysatoren bei erhöhter Temperatur und unter erhöhtem Druck und nachfolgender Behandlung mit molekularen Sauerstoff enthaltenden Gasen in wäßriger Essigsäure erhalten worden sind, wobei man

a) das Reaktionsgemisch in eine Kobaltacetat, Pyridin, Methylpyridine oder deren Gemische, Essigsäure und Methanol enthaltende wäßrige Phase und eine Butandicarbonsäuremethylester, Nebenprodukte und nicht umgesetzte Pentensäuremethylester, Pyridin, Methylpyridine oder deren Gemische sowie Essigsäure enthaltende organische Phase trennt.

b) die im Schritt a) erhaltene organische Phase mit wäßriger Essigsäure extrahiert und einen Pyridin oder Methylpyridine oder deren Gemische sowie Essigsäure enthaltenden wäßrigen Extrakt erhält.

c) den in Schritt b) erhaltenen wäßrigen Extrakt mit der wäßrigen Phase aus der Stufe a) vereinigt und

d) aus der in Stufe c) anfallenden wäßrigen Lösung Pyridin, Methylpyridine oder deren Gemische durch Destillation gewinnt und eine Lösung von Kobaltacetat in wäßriger Essigsäure erhält.

Das neue Verfahren hat den Vorteil, daß man Pyridin, Methylpyridine oder deren Gemische auf einfache Weise in einer wiederverwendbaren Form erhält und Kobaltacetatlösung in einer Form anfällt, die zur Herstellung von Kobaltcarbonylkatalysatoren geeignet ist.

Erfindungsgemäß geht man von Reaktionsgemischen aus, die durch Umsetzen von Pentensäuremethylester mit Kohlenmonoxid und Methanol in Gegenwart von Pyridin, Methylpyridinen oder deren Gemischen sowie Kobaltcarbonylkatalysatoren bei erhöhter Temperatur und unter erhöhtem Druck und nachfolgende Behandlung mit molekularen Sauerstoff enthaltenden Gasen in wäßriger Essigsäure erhalten worden sind. In der Regel geht man von Gemischen isomerer Pentensäuremethylester, insbesondere von 3-Pentensäuremethylester, aus. Vorteilhaft hält man bei der Umsetzung ein Molverhältnis von Pentensäuremethylester zu Methanol von 1:1,15 bis 4, eine Temperatur von 140 bis 200 °C, insbesondere 150 bis 180 °C und Drücke von 100 bis 400 bar ein. Kohlenmonoxid wird vorteilhaft im Überschuß z.B. bis zum 10fachen der stöchiometrischen Menge angewandt. Ferner hat es sich als vorteilhaft erwiesen, wenn je Mol Pentensäuremethylester 0,01 bis 0,08 mol Kobaltcarbonylkomplex sowie je Mol Kobaltkatalysator 2 bis 10 mol Pyridin oder Methylpyridine oder deren Gemische vorhanden sind. Nach dem Entspannen wird das erhaltene Reaktionsgemisch mit molekularem Sauerstoff oder solchen enthaltenden Gasen in wäßriger Essigsäure vorteilhaft bei einem pH-Wert von 3 bis 6 bei einer Temperatur von 80 bis 160 °C behandelt. Ein geeignetes Verfahren zur Herstellung des Reaktionsgemisches wird beispielsweise beschrieben in der EP-Anmeldung 10 581.

Das so erhaltene Reaktionsgemisch wird in eine Kobaltacetat, Pyridin oder Methylpyridine oder deren Gemische, Essigsäure und Methanol enthaltende wäßrige Phase und eine Butandicarbonsäuremethylester, Nebenprodukte, Essigsäure, Pyridin, Methylpyridine oder deren Gemische und nicht umgesetzte Pentensäuremethylester enthaltende organische Phase getrennt. Die Trennung erfolgt zweckmäßig nach üblichen Methoden, wie Dekantieren. Hierbei hält man zweckmäßig eine Temperatur von 0 bis 80° C ein.

Die in der Stufe a) anfallende organische Phase wird mit wäßriger Essigsäure extrahiert und ein Pyridin, Methylpyridine oder deren Gemische sowie Essigsäure enthaltender wäßriger Extrakt erhalten. Sofern in der zu extrahierenden organischen Phase nicht ausreichend Essigsäure enthalten ist, wird vorteilhaft dafür gesorgt, daß während der Extraktion durch Zugabe von weiterer Essigsäure je Mol Pyridin oder Methylpyridine oder deren Gemische jeweils mindestens 1 Mol Essigsäure vorliegt. Die Essigsäure kann bis zu einem Überschuß von 10 mol angewandt werden. Vorzugsweise wird die Extraktion im Gegenstrom in üblichen Extraktionsvorrichtungen wie Mixer-Settler, Siebbodenkolonnen mit oder ohne Pulsation, durchgeführt. Bei der Extraktion hält man zweckmäßig eine Temperatur von 10 bis 80° C ein und wendet vorteilhaft je kg organische Phase 1 bis 7 kg wäßrige Essigsäure an. Vorzugsweise verwendet man einen Teilstrom der in Stufe d anfallenden Cobaltacetat enthaltenden wäßrigen Essigsäure als Extraktionsmittel.

Besonders bewährt hat es sich, wenn man vor der Extraktion aus der organischen Phase durch Destillation Butandicarbonsäuremethylester als Sumpfprodukte abtrennt und für die Extraktion lediglich die Pyridin, Methylpyridine oder deren Gemische enthaltenden Leichtsieder, die Pentensäureester, Valeriansäureester und Isovaleriansäureester enthalten, verwendet.

In der Stufe c) werden der wäßrige Extrakt, der Essigsäure und Pyridin, Methylpyridine oder deren Gemische und gegebenenfalls Kobaltacetat enthält, mit der wäßrigen Phase aus der Stufe a), die Kobaltacetat, Pyridin und/oder Methylpyridine, deren Gemische, Essigsäure, Methanol sowie gegebenenfalls Methylacetat enthält, vereinigt. Zweckmäßig wird die wäßrige Phase aus der Stufe a) vorher mit einem geeigneten wasserunlöslichen Extraktionsmittel, wie Cyclohexan im Gegenstrom extrahiert und der nichtwäßrige Extrakt der organischen Phase der Stufe a) zugegeben.

Aus der in der Stufe c) resultierenden wäßrigen Lösung, die Kobaltacetat, Pyridin oder Methylpyridine oder deren Gemische, Essigsäure, Methanol und gegebenenfalls Methylacetat enthält, werden in Stufe d) Pyridin und/oder Methylpyridine durch Destillation gewonnen. Zweckmäßig trennt man zunächst die leichtsiedenden Anteile Methanol und Methylacetat über Kopf ab und erhält eine wäßrige Lösung als Sumpf, die Kobaltacetat, Essigsäure, Pyridin und/oder Methylpyridine enthält. Aus dieser Lösung gewinnt man vorzugsweise Pyridin, Methylpyridine als Azeotrop mit Wasser, während als Sumpf eine gereinigte wäßrige Lösung von Kobaltacetat und Essigsäure verbleibt. Vorteilhaft werden aus dem Azeotrop Wasser/Pyridin oder Methylpyridine oder deren Gemische mit einem wasserunlöslichen organischen Extraktionsmittel, wie Benzol, Toluol, Xylole oder deren Gemische extrahiert. Die verbleibende wäßrige Phase wird in die Azeotropdestillation zurückgeführt. Aus der erhaltenen organischen Phase, die Pyridin oder Methylpyridine oder deren Gemische, Extraktionsmittel sowie geringe Mengen Wasser enthält, werden restliches Wasser und Extraktionsmittel abdestilliert und als Sumpfprodukt wasserfreies Pyridin oder Methylpyridine oder deren Gemische erhalten.

Das Verfahren nach der Erfindung sei an folgendem Beispiel veranschaulicht.

Beispiel

Man geht von einem Reaktionsgemisch aus, das durch Umsetzen von 3-Pentensäuremethylester mit Methanol und Kohlenmonoxid in Gegenwart von Picolin und Kobaltcarbonyl und nachfolgender Behandlung mit Luft und wäßriger Essigsäure erhalten wurde. Dieses Gemisch wurde in einer Stufe a in eine wäßrige und organische Phase getrennt. Aus der organischen Phase wurden durch Destillation Butandicarbonsäuremethylester als Sumpfprodukte abgetrennt und die Leichtsieder wie folgt extrahiert (Stufe b).

Einer pulsierten Siebbodenkolonne wird bei 60° C 21,5 l/h eines organischen Penten- und Valerianesterstromes mit 13 % (m/m) β-Picolin und 3 % (m/m) freier Essigsäure zugeführt. Als Solvent werden im Gegenstrom 52 l/h einer wäßrigen Cobaltacetat/Essigsäure-Lösung (aus Stufe d) mit 5,2 % (m/m) freier Essigsäure geführt. Bei einer Pulsation von 120 Hüben/Minute und ca. 5 theoretischen Trennstufen wird in der organischen Lösung noch ein Restpicolingehalt von 0,7 % (m/m) analysiert, im Extrakt findet man 4,9 % (m/m) β-Picolin.

Der so erhaltene Extrakt wurde mit der wäßrigen Phase aus Stufe a) vereinigt (Stufe c) und in Stufe d) wie folgt aufgearbeitet.

Einer 40bödigen Kolonne wird auf den 28. praktischen Boden eine wäßrige, cobaltacethaltige Picolinlösung mit 80° C in einer Menge von 762 g/h zugeführt. Die Zusammensetzung lautet 2,8 % (m/m) β-Picolin, 0,18 % (m/m) Methanol, 3,7 % (m/m) freie Essigsäure und 0,1 % (m/m) 3-Pentenester; der Rest ist Wasser. Die Picolinkonzentration im Kopfdestillat beträgt 19,7 % (m/m) neben 0,6 % (m/m) 3-Pentenester und 3,4 % (m/m) Methanol. Dieser Kopfablauf von 135 g/h wird mit 13,3 g/h Toluol bei 70° C extrahiert. Man erhält 35 g/h Toluolphase mit einer Zusammensetzung von 56 % (m/m) β-Picolin, 25 % (m/m) Toluol, 15,1 % (m/m) Wasser, 2,1 % (m/m) Methanol und 1,8 % (m/m) 3-Pentenester.

Der wäßrige Rücklauf von 113,3 g/h enthält noch 7,5 % (m/m) β-Picolin, 3,3 % (m/m) Methanol und 0,06 % (m/m) Toluol. Die Kolonne wurde bei Normaldruck betrieben, die Kopftemperatur betrug 97°C, die Sumpftemperatur betrug 103°C. Im Sumpfablauf wurden noch 110 ppm β-Picolin gefunden.

In einer Destillationskolonne mit 9,4 m Sulzer CY-Packung werden 4,4 l/h eines Gemisches der Zusammensetzung von 65,3 % (m/m) Toluol, 27,6 % (m/m) β-Picolin, 4,2 % (m/m) 3-Pentensäureester, 0,9 % (m/m) 2-trans-Pentensäureester, 1,4 % (m/m) Wasser, 0,4 % (m/m) Valeriansäuremethylester und 0,2 % (m/m) Unbekannte mit 30°C auf eine Packungshöhe von 4,6 m, von unten gezählt, zugefahren. Mit 15 l/h Rücklauf (Toluolphase) von Raumtemperatur erhält man über Sumpf ein Produkt mit 89,8 % (m/m) β-Picolin, 4,5 % (m/m) 3-Pentenester, 4,8 % (m/m) 2-trans-Pentenester und einem Wassergehalt von < 0,02 % (m/m). Im Phasenscheider des Kopfprodukts findet man in der Toluolphase Picolinwerte < 0,1 % (m/m) neben ca. 1 % (m/m) Ester. Die Kolonne wurde bei Normaldruck betrieben. Es stellen sich eine Kopftemperatur von 110°C und eine Sumpftemperatur von 145°C ein.

**Patentansprüche**

1. Verfahren zur Rückgewinnung von Pyridin oder Methylpyridinen oder deren Gemischen aus Butandicarbonsäuremethylester enthaltenden Reaktionsgemischen, die durch Umsetzen von Pentensäuremethylester mit Kohlenmonoxid und Methanol in Gegenwart von Pyridin oder Methylpyridinen oder deren Gemischen sowie Kobaltkatalysatoren bei erhöhter Temperatur und unter erhöhtem Druck und nachfolgende Behandlung mit molekularen Sauerstoff enthaltenden Gasen in wäßriger Essigsäure erhalten worden sind, dadurch gekennzeichnet, daß man
a) das Reaktionsgemisch in eine Kobaltacetat, Pyridin, Methylpyridine oder deren Gemische, Essigsäure sowie Methanol enthaltende wäßrige Phase und eine Butandicarbonsäuremethylester, Nebenprodukte und nicht umgesetzte Pentensäuremethylester, Essigsäure sowie Pyridin oder Methylpyridine oder deren Gemische enthaltende organische Phase trennt.
b) die in der Stufe a) anfallende organische Phase mit wäßriger Essigsäure extrahiert und einen Pyridin oder Methylpyridine oder deren Gemische sowie Essigsäure enthaltenden wäßrigen Extrakt erhält.
c) den wäßrigen Extrakt aus der Stufe b) mit der wäßrigen Phase aus der Stufe a) vereinigt und
d) aus der gemäß Stufe c) erhaltenen wäßrigen Lösung Pyridin oder Methylpyridine oder deren Gemische durch Destillation gewinnt und eine wäßrige Kobaltacetat und Essigsäure enthaltende Lösung erhält.

2. Verfahren nach Anspruch 1, dadurch gekennzeichnet, daß man in der Extraktion gemäß Stufe b) je Mol Pyridin und/oder Methylpyridine mindestens 1 mol Essigsäure anwendet.

3. Verfahren nach den Ansprüchen 1 und 2, dadurch gekennzeichnet, daß man vor der Extraktion gemäß Stufe b) Butandicarbonsäuremethylester abtrennt.

4. Verfahren nach den Ansprüchen 1 bis 3, dadurch gekennzeichnet, daß man in der Stufe d) in einer ersten Destillation Methanol und Methylacetat abdestilliert und dann Pyridin oder Methylpyridine oder deren Gemische als Azeotrop mit Wasser gewinnt.

5. Verfahren nach den Ansprüchen 1 bis 4, dadurch gekennzeichnet, daß man aus dem in Stufe d) anfallenden Azeotrop Wasser/Pyridin und/oder Methylpyridine mit einem wasserunlöslichen organischen Extraktionsmittel Pyridin und/oder Methylpyridine extrahiert, die verbleibende wäßrige Phase in die Azeotropdestillation zurückführt und aus der organischen Phase Extraktionsmittel und restliches Wasser abdestilliert und als Sumpfprodukt wasserfreies Pyridin und/oder Methylpyridine erhält.

6. Verfahren nach den Ansprüchen 1 bis 5, dadurch gekennzeichnet, daß man in Stufe d) anfallende wäßrige Kobaltacetat und Essigsäure enthaltende Lösung in Stufe b) als Extraktionsmittel verwendet.

**Claims**

1. A process for recovering pyridine, methylpyridines or mixtures thereof from a methyl butanedicarboxylate-containing reaction mixture obtained by reacting methyl pentenoate with carbon monoxide and methanol in the presence of pyridine, methylpyridines or mixtures thereof and a cobalt carbonyl catalyst at elevated temperatures and under superatmospheric pressure and subsequent treatment with a molecular oxygen-containing gas in aqueous acetic acid, which comprises
a) separating the reaction mixture into on the one hand an aqueous phase containing cobalt acetate, pyridine, methylpyridines or mixtures thereof, acetic acid and methanol and on the other an organic phase containing methyl butanedicarboxylate, by-products, unconverted methyl pentenoate, acetic acid and also pyridine, methylpyridines or mixtures thereof,

b) extracting the organic phase obtained in step a) with aqueous acetic acid to obtain an aqueous extract containing pyridine or methylpyridines or mixtures thereof as well as acetic acid,

c) combining the aqueous extract obtained in step b) with the aqueous phase from step a) and

d) isolating pyridine, methylpyridines or mixtures thereof by distillation from the aqueous solution produced in step c) and obtaining a solution of cobalt acetate in aqueous acetic acid.

2. A process as claimed in claim 1, wherein in the extraction performed in step b) not less than 1 mole of acetic acid is employed per mole of pyridine and/or methylpyridines.

3. A process as claimed in claim 1 or 2, wherein methyl butanedicarboxylate is separated off before the extraction of step b).

4. A process as claimed in any of claims 1 to 3, wherein in step d) methanol and methyl acetate are distilled off in a first distillation and pyridine or methylpyridines or mixtures thereof are then isolated in the form of an azeotrope with water.

5. A process as claimed in any of claims 1 to 4, wherein the water/pyridine and/or methylpyridine azeotrope obtained in step d) is extracted with a water-insoluble organic extractant to remove pyridine and/or methylpyridines, the remaining aqueous phase is recycled into the azeotropic distillation to distill extractant and remaining water out of the organic phase and leaving anhydrous pyridine and/or methylpyridines as the bottom product.

6. A process as claimed in any of claims 1 to 5, wherein the aqueous cobalt acetate and acetic acid-containing solution obtained in step d) is used as an extractant in step b).

**Revendications**

1. Procédé pour la récupération de pyridine, de méthylpyridines ou de leurs mélanges à partir de mélanges réactionnels contenant de l'ester méthylique d'acide butanedicarboxylique, qui ont été obtenus par réaction d'ester méthylique d'acide penténique avec de l'oxyde de carbone et du méthanol en présence de pyridine, de méthylpyridines ou de leurs mélanges, ainsi que de catalyseurs à base de cobalt, à température élevée et sous pression élevée, suivie de traitement par des gaz contenant de l'oxygène moléculaire dans l'acide acétique aqueux, caractérisé en ce que

a) on sépare le mélange réactionnel en une phase aqueuse contenant de l'acétate de cobalt, de la pyridine, des méthylpyridines ou leurs mélanges, de l'acide acétique et du méthanol, et en une phase organique contenant de l'ester méthylique d'acide butanedicarboxylique, des produits secondaires et l'ester méthylique d'acide penténique n'ayant pas réagi, ainsi que de la pyridine, des méthylpyridines ou leurs mélanges,

b) on extrait à l'acide acétique aqueux la phase organique obtenue dans l'étape a) et on obtient un extrait contenant de la pyridine, des méthylpyridines ou leurs mélanges, ainsi que de l'acide acétique,

c) on réunit l'extrait aqueux provenant de l'étape b) et la phase aqueuse provenant de l'étape a), et

d) on récupère par distillation, à partir de la solution aqueuse obtenue dans l'étape c), la pyridine, les méthylpyridines ou leurs mélanges et on obtient une solution aqueuse contenant de l'acétate de cobalt et de l'acide acétique.

2. Procédé selon la revendication 1, caractérisé en ce qu'on utilise dans l'extraction de l'étape b), par mole de pyridine et/ou de méthylpyridines, au moins 1 mole d'acide acétique.

3. Procédé selon la revendication 1 ou 2, caractérisé en ce qu'avant l'extraction de l'étape b), on sépare l'ester méthylique d'acide butanedicarboxylique.

4. Procédé selon l'une quelconque des revendications 1 à 3, caractérisé en ce que dans l'étape d), on sépare le méthanol et l'acétate de méthyle dans une première distillation, puis on récupère la pyridine, les méthylpyridines ou leurs mélanges sous forme d'azéotrope avec de l'eau.

5. Procédé selon l'une quelconque des revendications 1 à 4, caractérisé en ce qu'à partir de l'azéotrope eau/pyridine et/ou méthylpyridines obtenu dans l'étape d), on extrait la pyridine et/ou les méthylpyridines avec un agent d'extraction organique insoluble dans l'eau, on renvoie la phase aqueuse résiduelle dans la distillation azéotrope, on sépare de la phase organique par distillation l'agent d'extraction et l'eau résiduelle et on obtient de la pyridine et/ou des méthylpyridines anhydres en tant que produit de bas de colonne.

6. Procédé selon l'une quelconque des revendications 1 à 5, caractérisé en ce qu'on utilise comme agent d'extraction dans l'étape b) la solution aqueuse contenant de l'acétate de cobalt et de l'acide acétique, obtenue dans l'étape d).